# EUROPÄISCHE PATENTSCHRIFT

(11) **EP 2 673 013 B1**
(45) Veröffentlichungstag und Bekanntmachung des Hinweises auf die Patenterteilung: **15.03.2017**
(21) Anmeldenummer: 12703688.7
(22) Anmeldetag: 26.01.2012
(51) Int. Cl.: A61L 27/24, A61L 27/52

(54) **GELIERENDES KOLLAGEN UND MITTEL ZU DESSEN BEREITSTELLUNG**
COAGULATING COLLAGEN AND MEANS FOR PREPARING SAME
COLLAGÈNE GÉLIFIANT ET MOYENS POUR LE METTRE À DISPOSITION

(30) Priorität: 09.02.2011 DE 102011011092
(43) Veröffentlichungstag der Anmeldung: 18.12.2013
(73) Patentinhaber: Amedrix GmbH, 73728 Esslingen (DE)
(72) Erfinder: GRAEVE, Thomas, 70597 Stuttgart (DE)
(74) Vertreter: Schrell, Andreas
(86) Internationale Anmeldenummer: PCT/EP2012/000336
(87) Internationale Veröffentlichungsnummer: WO 2012/107174

(56) Entgegenhaltungen:
- EP-B1- 1 221 937
- DE-A1-102005 034 420
- DE-A1-102006 026 591
- US-A1- 2003 211 793
- US-A1- 2009 254 104

## Beschreibung

Die Erfindung betrifft eine gefüllte Spritze zur Einbringung einer flüssigen gelierenden Kollagenzusammensetzung in die Kavität eines Knorpeldefekts, entsprechend dem Anspruch 1.

Im Zusammenhang mit degenerativen oder traumatischen Gelenkerkrankungen des menschlichen oder tierischen Körpers treten Knorpeldefekte in Form von fehlendem abgetragenem Knorpelmaterial auf. Geschädigter Gelenkknorpel zeigt nur eine beschränkte Fähigkeit zur Eigenregeneration. Zur Therapie ist es erforderlich, den Knorpeldefekt "aufzufüllen", um das fehlende Knorpelmaterial zu ersetzen. Dies erfolgt durch invasive chirurgische Maßnahmen. Bekanntermaßen werden Knorpeldefekte durch Transplantation knorpelbildender, insbesondere autologer, Zellen wie Chrondrozyten, in dem Defekt behandelt, wodurch dort die Neubildung von hyalinem Knorpel ausgelöst werden soll (Knorpeltransplantate). Andere, insbesondere zellfreie Verfahren setzen bestimmte Materialien und Materialzusammensetzungen ein, die als künstlicher Knorpel (Knorpelimplantat) dienen können. Bekannte Knorpelimplantate bilden eine Matrix oder ein Gerüst, in die Knorpelzellen des umgebenden intakten Knorpelgewebes einwandern können, um so eine neue knorpelige Struktur zu bilden.

Aus der DE 10 026 789 A1 sind eine Kollagen-basierte Biomatrix und Verfahren zu deren Herstellung bekannt. Es wird eine Kollagenbiomatrix aus Kollagen, das aus Rattenschwanzsehnen extrahiert wurde, hergestellt. Dazu wird die nach der Extraktion vorliegende saure Kollagenlösung in der Kälte mit serumhaltigem Puffer neutralisiert und, gegebenenfalls in Gegenwart von Zellen, zu einem Kollagengel gegossen, das später zu einer kollagenhaltigen Biomatrix geliert oder quervernetzt, das heißt aushärtet, die so als konfektioniertes zellfreies Kollagenimplantat oder zellhaltiges Kollagentransplantat mit eingebetteten Chrondrozyten erhalten werden kann.

EP 1221937 beschreibt ein Verfahren zur Herstellung eines unmittelbar gelierendes Kollagengels aus einer sauren Kollagenlösung und einer Pufferlösung, die getrennt voneinander bereitgestellt und temperiert werden können. Die Kollagenzusammensetzungen können in dem menschlichen / tierischen Körper durch eine Spritze eingebracht werden.

Aus US 2009/0254104 sind Kollagenzusammensetzungen mit hoher Kollagenkonzentration bekannt, die zusammen mit einem neutralisierenden Puffer vermischt werden können, um ein Kollagengel zu erzeugen. Das eingesetzte Kollagen ist ein Enzym-behandeltes Kollagen.

In bekannten chirurgischen Therapieverfahren werden die Knorpeldefekte zunächst freipräpariert, wobei gegebenenfalls bereits geschädigtes Knorpelgewebe gezielt entfernt wird. Es entsteht eine Kavität, die dann mit einem Knorpelimplantat gezielt gefüllt werden muss. Ein enger Kontakt zwischen Knorpelimplantat und dem umgebenden Gewebe ermöglicht das Einwandern von Knorpelzellen und damit eine Regeneration des Knorpels. Dazu müssen bekannte Knorpelimplantate, die konfektioniert bereitgestellt werden, während der Operation noch zusätzlich zurecht geschnitten und an die präparative Kavität angepasst werden. Besonders müssen die Ränder des Knorpelimplantats mit den Rändern der Kavität so in Eingriff kommen, dass eine stabile Verankerung des Implantats im Gelenkknorpel erzielt wird. Das Anpassen des konfektionierten Implantats an die Kavität ist aufwändig und zeitintensiv. Durch diese aufwändigen Maßnahmen erhöht sich das Risiko von Kontaminationen oder von Komplikationen bei der Operation. Weiter kann damit bei geringem operativem Aufwand eine vollständige Anpassung nicht erreicht werden; es besteht das Risiko von postoperativen Komplikationen oder des Ausbleibens des Behandlungserfolgs. Bisherige Verfahren und Mittel zur Therapie von Knorpeldefekten sind daher verbesserungswürdig.

Die Erfindung hat sich die Aufgabe gestellt, neuartige Mittel zur Herstellung einer kollagenbasierten Matrix bereitzustellen, die insbesondere zur Behandlung von Knorpeldefekten am menschlichen oder tierischen Körper eingesetzt werden können und wodurch die aus dem Stand der Technik bekannten Nachteile vermieden werden können.

Es wird auch ein Verfahren beschrieben, womit aus einer flüssigen konzentrierten Kollagenlösung in Verbindung mit einer flüssigen Pufferlösung eine unmittelbar gelierende Kollagenzusammensetzung erhalten wird. Diese kann bevorzugt direkt in die Kavität eines Knorpeldefektes injiziert werden, wo es unmittelbar zu einer Kollagenmatrix aushärtet, die das Knorpelimplantat dann direkt in situ bildet. Dabei ermöglicht die Einbringung der flüssigen gelierenden Kollagenzusammensetzung in die Kavität eine verbesserte Anpassung an die Form der Kavität, und die noch flüssige Kollagenzusammensetzung kann mit den Rändern der Kavität und deren Boden in direktem Kontakt kommen.

Das Verfahren enthält zumindest die folgenden Schritte: Getrennt voneinander bereitgestellte konzentrierte flüssige Kollagenlösung und flüssige Pufferlösung, die insbesondere zuvor in der Kälte gelagert waren, werden zusammen auf eine Temperatur von 20° C bis etwa 37° C, bevorzugt bis etwa 30° C, temperiert; temperierte Kollagenlösung und temperierte Pufferlösung werden, vorzugsweise unmittelbar daran anschließend, vermischt, wobei eine gelierfähige Kollagenzusammensetzung erhalten wird, die unmittelbar beginnt zu gelieren und zu einer Kollagenbiomatrix aushärten kann.

Besonders ist vorgesehen, dass Kollagenlösung und Pufferlösung erst bei der Applikation, das heißt besonders während der Applikation der Kollagenzusammensetzung vermischt werden und sich erst während der Applikation die zunächst noch flüssige gelierende Kollagenzusammensetzung bildet. Erfindungsgemäß wird also die gelierende aber noch flüssige Kollagenzusammensetzung in statu nascendi appliziert, die dann am Applikationsort aushärten kann.

Es werden eine konzentrierte Kollagenlösung und eine Pufferlösung bereitgestellt, die ungemischt, aber in einem integralen Gebinde zusammen lagerbar sind, und zwar in an sich bekannter Weise in der Kälte, besonders bei Temperaturen von etwa 0° C bis etwa 4° C. Im Rahmen des erfindungsgemäßen Verfahrens werden diese erst kurz vor deren Verwendung zur Herstellung der Kollagenbiomatrix temperiert, beispielsweise auf Zimmertemperatur, das heißt besonders auf 20° C oder mehr, oder auf Körpertemperatur, das heißt besonders etwa 30° C, nicht aber auf denaturierende Temperaturen von mehr als 37° C.

Besonders erfolgt die Temperierung von Kollagenlösung und Pufferlösung gleichzeitig, und zwar besonders erst unmittelbar vor dem erfindungsgemäßen Vermischen und Ausbringen. Dies kann durch kurzzeitiges Lagern in einem Wärmeschrank oder gegebenenfalls durch Aufwärmen in der Hand erfolgen. Die Erfindung sieht besonders vor, eine Temperatur der Kollagenzusammensetzung von mehr als 37° C zu vermeiden.

Erfindungsgemäß werden die temperierten Lösungen erst zu der oder für die Applikation in die Kavität oder einen anderen Ausbringungsort vermischt, um dabei die Kollagenzusammensetzung zu bilden, die unmittelbar bei der Applikation beginnt zu gelieren und in dem Applikationsort ausgeliert, das heißt aushärtet.

Sowohl Kollagenlösung als auch Pufferlösung liegen vor Gebrauch oder Applikation in flüssiger Form vor. Ihre niedrige Viskosität erlaubt vorteilhafterweise deren unmittelbare Vermischung ohne zusätzliche denaturierende Maßnahmen wie Erhitzen. Die dynamische Viskosität der Pufferlösung liegt bevorzugt im Bereich derer von Wasser oder leicht beweglichen wässrigen Lösungen, das heißt etwa 1 bis 5 mPa•s. Die dynamische Viskosität der konzentrierten Kollagenlösung liegt bevorzugt in der Größenordnung von etwa 10¹ bis 10⁵ mPa•s.

Kollagenlösung und Pufferlösung werden zunächst voneinander getrennt, bevorzugt in einer mehrkammerigen Spritze, bereitgestellt; sie werden besonders innerhalb der Spritze jeweils getrennt voneinander temperiert und beim Austrag aus der Spritze heraus unmittelbar vermischt. Besonders ist vorgesehen, dass Zusammenbringen und Vermischen von Kollagenlösung und Pufferlösung durch Ausdrücken der Lösungen aus den Kammern der Spritze und durch Zusammenführen der Lösungsströme innerhalb der Spritze in einer der Spritze zugeordneten Mischvorrichtung erfolgt, wobei die frisch hergestellte Kollagenzusammensetzung aus dem Ausgang der Mischvorrichtung austritt.

Die Erfindung erlaubt die Herstellung einer kollagenhaltigen Biomatrix oder eines Kollagenimplantats mit einem hohen Anteil an nicht denaturiertem, quervernetzbarem Kollagen nativer Struktur. Es hat sich gezeigt, dass Knorpelzellen besonders gut in einer Kollagenbiomatrix proliferieren und dabei eine eigene Kollagensynthese mit hoher Rate aufweisen, wenn in der Biomatrix ein hoher Anteil an nativem Kollagen enthalten ist. Die Erfindung vermeidet daher kollagendenaturierende Maßnahmen.

Die Erfindung sieht bevorzugt vor, dass die konzentrierte Kollagenlösung unmittelbar und ohne denaturierende Schritte aus kollagenhaltigem Gewebe gewonnen wird. Ein bevorzugtes kollagenhaltiges Gewebe sind präparierte Rattenschwanzsehnen. Daraus wird das Kollagen bevorzugt mittels saurer, besonders essigsaurer, Extraktion gewonnen. Als konzentrierte Kollagenlösung wird eine saure Kollagenlösung bereitgestellt, die einen Kollagengehalt (Kollagenkonzentration) von mehr als 8 mg/ml, besonders von etwa 9 mg/ml und mehr oder bevorzugt bis etwa 16 mg/ml aufweist. Die konzentrierte Kollagenlösung ist vor allem sauer, um deren Viskosität zu erhalten. Dabei beträgt der pH-Wert der konzentrierten Kollagenlösung (bezogen auf 21° C) pH 6 oder weniger, besonders von pH 5 bis pH 3,5.

In einer besonderen Ausgestaltung enthält die konzentrierte Kollagenlösung keine weiteren Zusätze oder Hilfsstoffe wie Zellen, Zellbestandteile, Wachstumsfaktoren wie Cytokine, Immunstimulanzien, Antibiotika oder Stabilisierungsmittel wie Polysaccharide. In alternativer Ausgestaltung ist in der Kollagenlösung mindestens ein solcher Zusatz- oder Hilfsstoff enthalten.

Um die gelierende Kollagenzusammensetzung zu erhalten, wird die konzentrierte saure Kollagenlösung mit einem neutralisierenden Puffer vermischt. Der neutralisierende Puffer ist im einfachsten Fall eine an sich bekannte Puffersalzlösung, wodurch der pH-Wert der Kollagenzusammensetzung auf einen neutralen Bereich, insbesondere pH 7,0 bis pH 7,5 (bezogen auf 21° C), erreicht wird. Bevorzugt wird eine HEPES-gepufferte Saline mit pH 8,3 eingesetzt, die in an sich bekannter Weise herstellbar ist. Die Pufferlösung dient erfindungsgemäß außerdem dazu, die konzentrierte Kollagenlösung zu verdünnen, um die Endkonzentration in der gelierenden Kollagenzusammensetzung zu erzielen. Die Pufferzusammensetzung ist je nach vorgesehenem Verhältnis der Mischung mit der konzentrierten Kollagenlösung bevorzugt zumindest zweifach konzentriert (bei 1+1) und bevorzugt maximal zehnfach konzentriert (bei 9+1).

In einer besonderen Ausgestaltung enthält die Pufferlösung keine weiteren Zusätze oder Hilfsstoffe wie Zellen, Zellbestandteile, Wachstumsfaktoren wie Cytokine, Immunstimulanzien oder Stabilisierungsmittel wie Polysaccharide. In alternativer Ausgestaltung sind in der Pufferlösung Zellen und gegebenenfalls mindestens ein weiterer Zusatz- oder Hilfsstoff enthalten, die bei dem erfindungsgemäßen Vermischen mit der konzentrierten Kollagenlösung eine zellhaltige gelierende Kollagenzusammensetzung bilden, die zu einem zellhaltigen Kollagentransplantat aushärtet. In einer besonderen Variante davon sind die Zellen Chrondrozyten, vor allem autologe Zellen und Stammzellen.

Bevorzugt ist vorgesehen, Kollagenlösung und Pufferlösung in einem Volumenverhältnis von 1+1 bis 9+1 (Kollagen- zu Pufferlösung) zu vermischen. Bevorzugt sind Mischungsverhältnisse von 4+1, das heißt von vier Teilen Kollagenlösung zu einem Teil Pufferlösung. Demgemäß beträgt der Kollagengehalt in der hergestellten Kollagenzusammensetzung bevorzugt mindestens 6 mg/ml oder mehr, besonders von 6 bis 12 mg/ml. In einer bevorzugten Variante davon beträgt der Gehalt in der Kollagenzusammensetzung etwa 8 mg/ml.

Es wird eine mehrkammerige Spritze eingesetzt. Diese ist besonders eine an sich bekannte Einmalspritze. Die Spritze ist mit einem an sich bekannten statischen Mischer als Mischvorrichtung ausgestattet. Der Fachmann kennt daneben andere integrale Mischvorrichtungen, die ein Vermischen von getrennten Lösungen bei der Applikation ermöglichen.

Werden Kollagenlösung und Pufferlösung in einer mehrkammerigen Spritze bereitgestellt, weist diese besonders jeweils Kammervolumina von etwa 0,5 bis etwa 5 ml auf. Dabei findet das erfindungsgemäße Temperieren der Lösungen innerhalb der Spritze statt, ebenso das Vermischen von Kollagenlösung und Pufferlösung beim Austragen der Lösung aus der Spritze. Bevorzugt ist vorgesehen, dass die Mischvorrichtung der Spritze ein an sich bekannter statischer Mischer ist. Der Fachmann kennt alternative Mischvorrichtungen, die im Zusammenhang mit Spritzen eingesetzt werden können. Der Vorgang des Ausbringens soll je nach Spritzenvolumen und Ausbringgeschwindigkeit etwa 1 bis 60 Sekunden dauern. Die Vermischdauer beträgt erfindungsgemäß für jeden aus den beiden Kammern austretenden infinitesimalen Volumenteil bevorzugt etwa 0,5 bis etwa 2 Sekunden.

Die naszierende Zusammensetzung beginnt unmittelbar zu gelieren und zwar besonders innerhalb von 10 Sekunden nach dem Vermischen, bevorzugt innerhalb von 5 Sekunden. Die gelierende Zusammensetzung ist noch flüssig und vor allem noch fließfähig. Der Geliervorgang ist abgeschlossen, wenn die Kollagenzusammensetzung zu einer festen Biomatrix aushärtet. Das Aushärten zu einer Biomatrix ist bevorzugt innerhalb von 2 bis 4 Minuten abgeschlossen. Beispielsweise findet so die Aushärtung der in die Kavität des Knorpeldefektes applizierten zunächst noch fließfähigen Kollagenzusammensetzung erst in der Kavität selbst statt, sodass dort ein unmittelbar an die Kavität angepasstes Knorpelimplantat entsteht.

Dadurch, dass das verfahren erlaubt, die hergestellte Kollagenzusammensetzung noch in flüssiger Form an den Applikationsort, beispielsweise die Kavität, zu transportieren, sind weniger große Zugänge an dem Situs erforderlich. Dies begünstigt die Operation im Rahmen eines athroskopischen oder endoskopischen Zugangs. Das bisher notwendige Transportieren eines bekannten bereits ausgehärteten konfektionierten Knorpelimplantats durch einen endoskopischen Zugang an den Situs entfällt. Die Erfindung erlaubt eine verbesserte anthroskopische beziehungsweise minimal-invasive Chirurgie und verringert so die durch den operativen Eingriff verursachten Traumata.

Die Erfindung stellt eine fertig gefüllte Spritze, insbesondere in Form einer Einmalspritze als Kit, bereit, wobei die Spritze mindestens zwei getrennte Kammern enthält, die in einer der Spritze zugeordneten Spritzvorrichtung münden, durch die der Inhalt jeder Kammer bevorzugt simultan abgegeben werden kann. Erfindungsgemäß ist die Spritze zumindest dadurch gekennzeichnet, dass mindestens eine erste Kammer mit der flüssigen konzentrierten Kollagenlösung und mindestens eine davon getrennte zweite Kammer mit der Pufferlösung gefüllt sind. Daneben können weitere solche Kammern an der Spritze vorgesehen sein, die mit Zusatz- oder Hilfsstoffen gefüllt sein können. Die zumindest mit erfindungsgemäßer Kollagenlösung und Pufferlösung gefüllte Spritze stellt ein Kit dar, das zur Herstellung einer unmittelbar gelierenden Kollagenzusammensetzung eingesetzt werden kann.

Es sind andere Ausgestaltungen denkbar, die eine getrennte Lagerung von konzentrierter Kollagenlösung und Pufferlösung und die anschließende unmittelbare Vermischung dieser Lösungen zur Herstellung der gelierenden Kollagenzusammensetzung erlauben. Eine alternative Ausgestaltung ist insbesondere eine mehrkammerige Mischkapsel zur einmaligen Verwendung, die im Zusammenhang mit einem an sich bekannten Kapselmischer eingesetzt werden kann.

Beschrieben wird auch die Verwendung der gefüllten Spritze zur Anwendung zur medizinischen Therapie. Eine besondere Anwendung ist die prophylaktische oder therapeutische Behandlung von Knorpeldefekten des menschlichen oder tierischen Körpers. Diese Knorpeldefekte treten insbesondere an Gelenken auf. Die Verwendung ist jedoch nicht auf Gelenkknorpeldefekte beschränkt. Die Erfindung kann vielmehr zur Behandlung weiterer Knorpeldefekte und anderer Gewebedefekte des menschlichen oder tierischen Körpers eingesetzt werden.

Andere medizinische Anwendungen der erfindungsgemäßen gefüllten Spritze umfassen die Therapie eines Gewebeverlusts im Nukleus nach Bandscheibenvorfall und die Behandlung von Weichteildefekten in der Haut, von Knochen- und Sehnendefekten sowie den Aufbau von Zahnfleischdefekten und anderen Anwendungen in der Zahn- und Kieferheilkunde sowie Anwendungen in der plastischen Chirurgie, besonders wenn es um Auffüllen von Defekten oder Kavitäten geht.

Ein weiterer Aspekt ist die Verwendung der erfindungsgemäß gefüllten Spritze zur Herstellung einer kollagenhaltigen Biomatrix für die Gewebekultur. In einer besonderen Ausgestaltung kann mittels des Verfahrens eine Biomatrix für Zellkultur unmittelbar hergestellt werden. Besonders ist dies für die Herstellung von Sandwichkulturen geeignet, worin Zellen oder Gewebe mit einer kollagenhaltigen Biomatrix überschichtet werden sollen, um die Zellen oder Gewebe darin einzubetten. Neben anderer medizinischer ist auch eine Vielzahl nichtmedizinischer Anwendungsmöglichkeiten gegeben, in den einfache und zuverlässige Herstellung eines unmittelbar gelierenden Kollagengels gefordert wird.

Die Erfindung wird durch die Figuren und die nachfolgenden Ausführungsbeispiele näher beschrieben, ohne dass diese beschränkend zu verstehen wären.

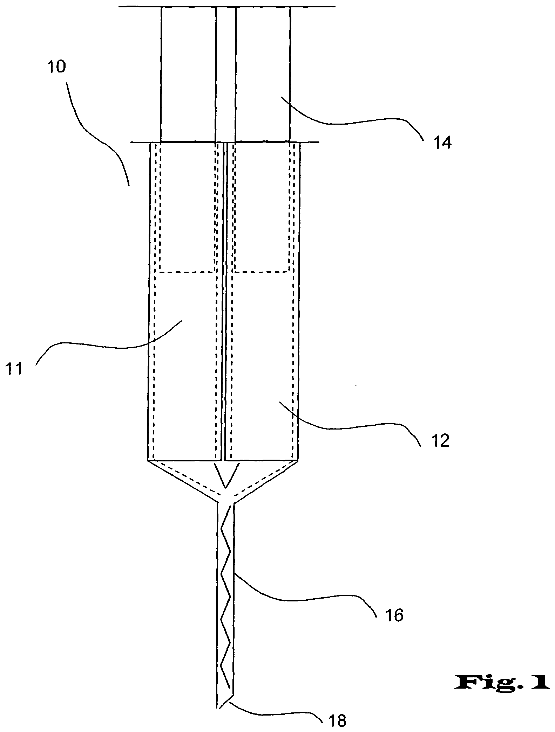

Figur 1 zeigt eine schematische Darstellung einer Ausgestaltung der erfindungsgemäßen gefüllten Spritze.

In der dargestellten Ausführung sind in einem integralen Behälter zwei getrennte parallele zylindrische Kammern (11, 12) zur Aufnahme der Kollagenlösung einerseits und Pufferlösung andererseits ausgebildet. Beide Kammern münden vorderseits in einen gemeinsamen Austrittskanal (18), der einen statischen Mischer (16) mit Mischschikane aufweist. Zum Entleeren der Kammern (11, 12) zur Applikation und Herstellung der Kollagenzusammensetzung sind gekoppelte Spritzenstempel (14) vorgesehen. Diese bilden die rückseitige Begrenzung des Volumens der Kammern: in an sich bekannter Weise kann durch Druckbeaufschlagung der Stempel (14) der Inhalt simultan aus beiden Kammern (11, 12) ausgedrückt werden. Je nach Verhältnis des baulich vorgegebenen Volumens der Kammern (11, 12) findet beim Ausdrücken eine gleichmäßige Vermischung der sich in den Kammern (11, 12) befindlichen Flüssigkeit in diesem Volumenverhältnis statt. In der dargestellten Ausgestaltung ist das Volumenverhältnis der Kammern (11, 12) 1:1.

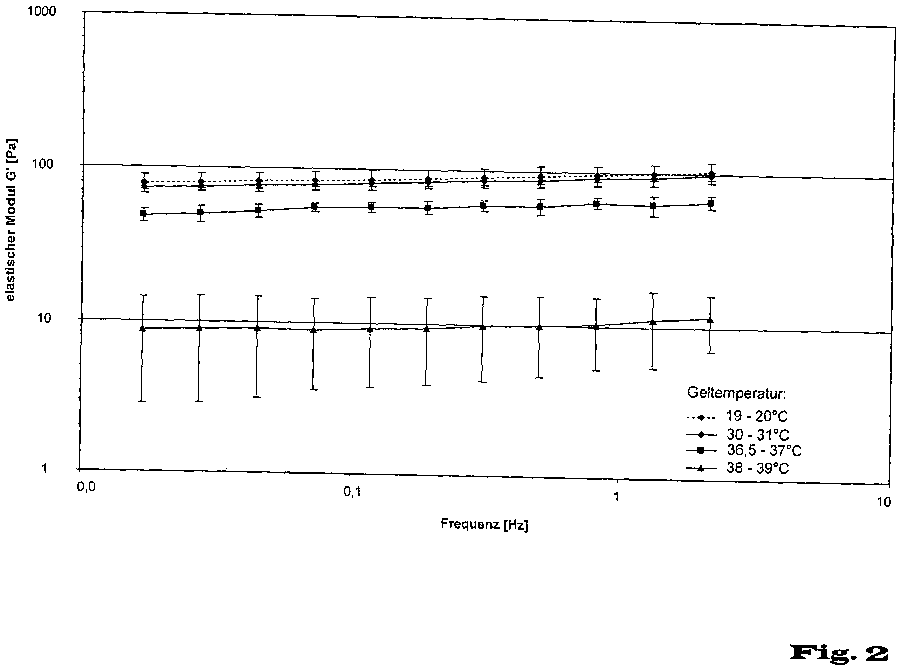

Figur 2 zeigt die Ergebnisse rheologischer Untersuchungen an Kollagengelen: elastischer Modulus in Abhängigkeit von der Testfrequenz (Mittelwerte und Standardabweichungen) bei unterschiedlich vortemperierten Zweikammerspritzen.

### Beispiel 1: Herstellung einer unmittelbar gelierenden Kollagenzusammensetzung

Zur Herstellung einer konzentrierten Kollagenlösung werden Rattenschwänze bei etwa minus 20° C gelagert und anschließend für einige Minuten in etwa 70 %igem Alkohol oberflächlich desinfiziert. Es wird die Haut abgezogen und die einzelnen Kollagenfasern herausgelöst. Die Kollagenfasern werden nochmals in Alkohol oberflächlich desinfiziert, danach mit PBS gewaschen, anschließend in eine etwa 0,1 %ige (0,5 mol/l) Essigsäurelösung überführt und dort inkubiert. Die Kollagenfasern werden in der Essigsäurelösung für einen Zeitraum von mindestens 7 Tagen in der Kälte (bei etwa 0 bis 4° C) gerührt. Nach Abtrennen der nichtgelösten Kollagenteile am Ende der Inkubationszeit wird das Kollagen filtriert und ausgefällt. Das Präzipitor wird mit Pufferlösung gespült, eingefroren und anschließend gefriergetrocknet. Das gefriergetrocknete Kollagen wird in 0,1 % Essigsäure definiert aufgenommen, sodass ein Kollagengehalt von 9 bis 16 mg/ml erhalten wird. Der pH-Wert der konzentrierten Kollagenlösung beträgt etwa 4,0.

Zur Vermischung von vier Teilen Kollagenlösung mit einem Teil neutralisierender Pufferlösung (4+1) wird eine fünffach konzentrierte Pufferlösung angesetzt. Als fünffach konzentrierte Pufferlösung wird dazu insbesondere eine Lösung von 35,6 g NaCl in 937,5 ml Reinstwasser mit 62,5 ml 3 mol/l HEPES-Lösung angesetzt. Vor Gebrauch wird die Pufferlösung mit NaOH auf pH 8,3 eingestellt.

Kollagenlösung und Pufferlösung werden in jeweils getrennte Kammern einer Mehrkammerspritze, die ein Kammervolumenverhältnis von 1:4 aufweist, eingefüllt und bis zu deren weiteren Verwendung in der Kälte, bei etwa -15° C oder kälter, gelagert.

Zur Herstellung einer kollagenhaltigen Biomatrix wird die Mehrkammerspritze kurzfristig in einen Wärmeschrank oder ein Wasserbad gelegt, wodurch Pufferlösung und Kollagenlösung auf eine Temperatur von etwa 30° C, erwärmt werden. Zum Vermischen der beiden Lösungen werden diese über die gekoppelten Spritzenkolben aus der Mehrkammerspritze gedrückt. Dabei werden beide Lösungen über die mit den Kammern verbundene Mischvorrichtung geführt. Aus der Spritze tritt die vermischte, unmittelbar gelierende Kollagenzusammensetzung aus. Diese wird in noch flüssiger Form in eine Gießform eingefüllt. Nach dem Ausbringen füllt die Kollagenzusammensetzung die Gießform völlig aus, und innerhalb weniger Minuten härtet sie zu einer festen kollagenhaltigen Biomatrix aus. Bei einem Kollagengehalt der durch die Vermischung entstehenden Kollagenzusammensetzung von etwa 8 mg/ml und einer Temperatur von etwa 30° C härtet diese innerhalb von 2 Minuten vollständig aus.

### Beispiel 2: Gelierung einer Kollagenzusammensetzung

Die gemäß Beispiel 1 herstellbaren Kollagenzusammensetzungen werden rheologischen Untersuchungen unterzogen. In Zweikammerspritzen (z. B. Firma Medmix Systems, CH) werden Kollagenlösung (10 mg/ml) und Pufferlösung gemäß Beispiel 1 gefüllt (Kollagenkammer: 4 Teile Kollagenlösung, 2 ml; Pufferkammer: 1 Teil fünffach konzentrierter Puffer, 0,5 ml) und eingefroren.

Dem einstündigen, schonenden Auftauen bei Raumtemperatur (20,5 °C) folgt eine zehnminütige Temperierung der Spritzen im Wasserbad bei unterschiedlichen Temperaturen im Bereich von 20 bis 40 °C (Tabelle 1).

**Tabelle 1:**

| Temperatur Soll [°C] (Temperaturbereich Ist [°C]) | | Anzahl der unter-suchten Spritzen |
|---|---|---|
| 20 | (19 - 20) | 3 |
| 30 | (30 - 31) | 4 |
| 37 | (36,5 - 37) | 4 |
| 38 | (38 - 39) | 3 |
| 40 | (40 - 41) | 4 |

Zum Mischen beider Komponenten nach der Temperierung wird der Verschluss der Spritze durch einen Mischadapter ausgetauscht und der Inhalt vorsichtig in ein Well einer 12-Well-Gewebekulturplatte abgegeben, dabei werden die ersten zwei austretenden Tropfen verworfen.

Die vollständige Gelierung der Kollagenzusammensetzung (8 mg/ml Kollagen), das heißt Aushärtung zu einem festen Gel, erfolgte in 15 min bei 20,5 °C. Anschließend erfolgt eine visuelle Beurteilung der Konsistenz (Tabelle 2).

Anschließend werden die elastischen Module der Gele außerhalb des Wells durch Frequenzverfahren ermittelt (Bohlin CVO R 150, Fa. Malvern Instruments GmbH, DE) (Figur 2).

**Tabelle 2:**

| Temperatur Soll [°C] (Temperaturbereich Ist [°C]) | | Konsistenz der Gele nach 15 min |
|---|---|---|
| 20 | (19 - 20) | fest |
| 30 | (30 - 31) | fest |
| 37 | (36,5 - 37) | fest |
| 38 | (38 - 39) | halbfest |
| 40 | (40 - 41) | flüssig |

Figur 2 ist zu entnehmen, dass die höchsten ermittelten Mittelwerte des elastischen Moduls in einem Temperaturbereich von 20 und 30 °C liegen. Die Kurve des auf 37 °C erwärmten Kollagens verläuft etwas tiefer, jedoch haben auch diese Gele bei visueller Betrachtung eine feste Konsistenz gezeigt. Bei Erwärmung auf 38 °C waren die Gele nur halbfest und wiesen bei der Oszillationsmessung sehr niedrige Werte mit großen Schwankungen auf. Das 40 °C warme Kollagen geliert nicht mehr.

## Patentansprüche

1. Gefüllte Spritze zur Einbringung einer flüssigen gelierenden Kollagenzusammensetzung in die Kavität eines Knorpeldefekts, enthaltend mindestens zwei getrennte Kammern, die in einen der Spritze zugeordneten statischen Mischer münden, durch den der Inhalt jeder Kammer simultan gemischt und abgegeben werden kann, wobei die mindestens eine erste Kammer mit einer flüssigen Lösung nativen Kollagens mit einer Konzentration von mehr als 8 mg/ml bis 16 mg/ml gefüllt ist, welche einen pH-Wert (bezogen auf 21 °C) von 6 oder weniger aufweist und mindestens eine davon getrennte zweite Kammer mit einer neutralisierenden Pufferlösung gefüllt ist.

2. Spritze nach Anspruch 1, wobei die aus der Spritze abgegebene Kollagenzusammensetzung eine Kollagenkonzentration von 6 mg/ml oder mehr aufweist.

## Claims

1. A filled syringe for introducing a liquid gelling collagen composition into the cavity of a cartilage defect, containing at least two separate chambers that open into a static mixer associated with the syringe, through which the content of each chamber can be mixed and dispensed simultaneously, wherein the at least one first chamber is filled with a liquid solution of native collagen having a concentration greater than 8 mg/ml to 16 mg/ml, wherein the pH of the collagen solution (based on a temperature of 21 °C) is 6 or less, and at least one separate second chamber is filled with a neutralizing buffer solution.

2. Syringe according to claim 1, wherein the collagen composition dispensed of the syringe has a collagen concentration of 6 mg/ml or greater.

## Revendications

1. Seringue chargée destinée à introduire une composition de collagène gélifié liquide dans la cavité d'un défaut de cartilage, comprenant au moins deux chambres séparées qui débouchent dans un mélangeur statique associé à la seringue et qui permet de mélanger et d'administrer simultanément le contenu de chaque chambre, dans laquelle l'au moins une première chambre est chargée d'une solution liquide de collagène natif qui a une concentration de plus de 8 mg/ml à 16 mg/ml et présente une valeur pH de 6 ou moins (par rapport à 21 °C) et au moins une seconde chambre séparée de celle-ci est chargée d'une solution tampon neutralisante.

2. Seringue selon la revendication 1, dans laquelle la composition de collagène administrée par la seringue présente une concentration de collagène de 6 mg/ml ou plus.
